# EUROPÄISCHE PATENTSCHRIFT

(11) **EP 3 134 726 B1**
(45) Veröffentlichungstag und Bekanntmachung des Hinweises auf die Patenterteilung: **22.11.2023**
(21) Anmeldenummer: 15723148.1
(22) Anmeldetag: 27.04.2015
(51) Int. Cl.: G01N 21/89, G01N 33/38, G01N 21/896, G01N 21/958

(54) **VERFAHREN UND VORRICHTUNG ZUM ERKENNEN VON NICKELSULFID-EINSCHLÜSSEN IN EINER GLASPLATTE**
METHOD AND DEVICE FOR DETECTING NICKEL SULPHIDE INCLUSIONS IN A GLASS PLATE
PROCÉDÉ ET DISPOSITIF PERMETTANT DE DÉTECTER DES INCLUSIONS DE SULFURE DE NICKEL DANS UNE PLAQUE DE VERRE

(30) Priorität: 25.04.2014 DE 102014005932
(43) Veröffentlichungstag der Anmeldung: 01.03.2017
(73) Patentinhaber: HEGLA boraident GmbH & Co. KG, 37688 Beverungen (DE); Technische Universität Darmstadt, 64289 Darmstadt (DE); Hilcken, Niklas, 60486 Frankfurt am Main (DE)
(72) Erfinder: HILCKEN, Niklas, 60486 Frankfurt am Main (DE); SCHNEIDER, Jens, 64285 Darmstadt (DE); HILCKEN, Jonas, 60385 Frankfurt am Main (DE); REDMANN, Frank, 06193 Petersberg (DE); RAINER, Thomas, 38855 Wernigerode (DE)
(74) Vertreter: Habermann Intellectual Property Partnerschaft von Patentanwälten mbB
(86) Internationale Anmeldenummer: PCT/EP2015/059103
(87) Internationale Veröffentlichungsnummer: WO 2015/162303

(56) Entgegenhaltungen:
- EP-A1- 1 839 043
- WO-A1-94/08229
- WO-A1-2009/031420
- WO-A1-2012/077683
- JP-A- 2001 089 174
- JP-A- 2010 249 552
- US-B1- 6 236 734
- US-B2- 7 511 807
- LI X ET AL: "Inspection and Image Analysis of Nickel Sulphide Inclusions in Toughened Glass Panels", CONTROL, AUTOMATION, ROBOTICS AND VISION, 2006. ICARCV '06. 9TH INTERN ATIONAL CONFERENCE ON, IEEE, PI, 5. Dezember 2006 (2006-12-05), Seiten 1-6, XP031334160, ISBN: 978-1-4244-0341-7 -& Xiang et al.: "In-Situ Inspection of Inclusions in Toughened Glass Panels of High-Rise Buildings", , Bd. 10, Nr. 4 30. April 2005 (2005-04-30), Seiten 1/7-7/7, XP002742907, NDT.net Gefunden im Internet: URL:http://www.ndt.net/article/icem2004/pa pers/248/248.htm [gefunden am 2015-07-28]

## Beschreibung

Die Erfindung betrifft ein Verfahren sowie eine Vorrichtung zum Erkennen von Nickelsulfid-Einschlüssen in einer Glasplatte.

Bei der Herstellung von Glasplatten treten unvermeidbar eine Anzahl von räumlich begrenzten Fehlstellen wie beispielsweise Verunreinigungen oder Defekte auf, durch welche die Qualität und Güte der Glasplatte beeinträchtigt werden können. So können beispielsweise während der Herstellung Blasen im Inneren der Glasplatte oder an einem Randbereich der Glasplatte entstehen. Weiterhin können während der Herstellung Verunreinigungen wie beispielsweise Staubpartikel, Verschmutzungen oder Oberflächendefekte nicht ausgeschlossen werden, selbst wenn der Herstellungsprozess mit großer Sorgfalt und mit einem erheblichen Aufwand an die Reinheit der für die Herstellung verwendeten Komponenten und die Umgebungsbedingungen durchgeführt wird. Zudem kommt es während der Produktion von Glasplatten immer wieder vor, dass einzelne Partikel in der Glasplatte eingeschlossen werden.

Die meisten lokalen Defekte bewirken lediglich optische und ästhetische Beeinträchtigungen der Glasplatte und gefährden die mechanische Stabilität der Glasplatte nicht. Es hat sich jedoch gezeigt, dass Nickelsulfid-Einschlüsse das Risiko eines plötzlich auftretenden und nicht vorhersehbaren Spontanbruchs der Glasplatte erheblich erhöhen. Insbesondere bei großflächigen Glasplatten, die beispielsweise als Glasscheiben zur Verkleidung von Gebäudefassaden oder in Glasfronten bzw. als großflächige Fensterscheiben verwendet werden, stellen die ohne frühzeitige Ankündigung erst nach Monaten oder Jahren auftretenden Spontanbrüche und die dadurch bewirkten Zerstörungen von Glasplatten ein erhebliches Gefährdungsrisiko dar. Glasplatten können auch in Fahrzeugen oder Maschinen verwendet werden und verschiedene Zusammensetzungen sowie Beschichtungen oder Nachbehandlungen bzw. Veredelungen aufweisen.

Untersuchungen haben gezeigt, dass die im Glas eingeschlossenen Nickelsulfid-Einschlüsse eine verzögerte Phasenumwandlung durchmachen. Hierdurch vergrößert sich deren Volumen. Der dadurch bewirkte lokale Spannungsanstieg kann bei Glasplatten und insbesondere bei thermisch vorgespannten Glasplatten, die beispielsweise als Einscheibensicherheitsglas eingesetzt werden, zu einem unangekündigten Spontanbruch führen.

Um die Gefahr von Personenschäden oder Sachschäden durch derartige Glasbrüche zu reduzieren, dürfen üblicherweise nur solche thermisch vorgespannten Glasscheiben eingesetzt werden, die vorher einem normierten Heißlagerungstest unterzogen wurden. Dabei wird die zu testende Glasplatte nach deren Herstellung auf etwa 280°C - 300°C erhitzt und für mehrere Stunden auf dieser Temperatur gehalten. Eine derart hohe Temperaturbelastung führt fast immer zu einem Zerbersten der Glasplatte, falls diese einen Nickelsulfid-Einschluss aufweist. Der Heißlagerungstest hat zu einer deutlichen Reduzierung des Gefährdungspotenzials durch Nickelsulfid-Einschlüsse in Glasplatten geführt. Allerdings ist der Heißlagerungstest energieaufwändig, verursacht einen logistischen und zeitlichen Mehraufwand während und nach dem Herstellungsprozess und führt üblicherweise zu einer Zerstörung der Glasplatte, falls diese einen Nickelsulfid-Einschluss aufweist.

In dem europäischen Patent EP 1 839 043 B1 wird ein Verfahren zur Prüfung von Glasscheiben auf gegebenenfalls vorhandene Nickelsulfid-Einschlüsse beschrieben. Dabei werden die Glasscheiben auf mindestens 500°C aufgeheizt und die Nickelsulfid-Einschlüsse anhand der von den Einschlüssen emittierten Strahlung identifiziert. Es hat sich jedoch gezeigt, dass die Nickelsulfid-Einschlüsse nicht mit ausreichender Genauigkeit von anderen Einschlüssen oder lokalen Defekten unterschieden werden können. Dieses Prüfungsverfahren ermöglicht deshalb nicht die für eine industrielle Nutzung und wirtschaftliche Umsetzung erforderliche Zuverlässigkeit bei der Erkennung von Nickelsulfid-Einschlüssen.

In der US-Patentschrift US 7 511 807 B2 wird ein Verfahren beschrieben, mit welchem Einschlüsse in einer Glasplatte durch das Streulicht eines über ein lichtleitendes Medium in die Glasplatte eingekoppelten Lichtstrahls sichtbar gemacht werden können. Eine Unterscheidung verschiedener Einschlüsse und insbesondere eine zuverlässige Erkennung von Nickelsulfid-Einschlüssen sind mit diesem Verfahren allerdings ebenfalls nicht erfolgversprechend möglich.

Bei einem weiteren Verfahren, das beispielsweise in der US-Patentschrift US 6,236,734 B1 beschrieben ist, werden mit einer Farbkamera Glasplatten in Fassaden oder Fensterscheiben fotografiert und die mit der Farbkamera erzeugten Bilder ausgewertet. Bei der Auswertung wird eine Unterscheidung der verschiedenen Einschlüsse anhand der in den Bildern wiedergegebenen Farbgebung der Einschlüsse versucht. Es hat sich jedoch gezeigt, dass die Farbe von Nickelsulfid-Einschlüssen in Abhängigkeit von der jeweiligen chemischen Zusammensetzung, aber auch in Abhängigkeit von den Umgebungsbedingungen und Parametern bei der Aufnahme des Farbbildes mit der Farbkamera deutliche Unterschiede aufweisen kann und deshalb keine eindeutige und zuverlässige Erkennung von Nickelsulfid-Einschlüssen möglich ist.

In JP 2010 249552 A ist ein Verfahren beschrieben, bei dem bei einer schräg auf die Glasplatte einfallenden Beleuchtung mit einer einzigen Kamera zwei verschiedene Abbildungen erzeugt und anhand eines räumlichen bzw. bei einer bewegten Glasplatte anhand eines zeitlichen Versatzes zwischen zwei festgestellten Abschattungen auf einen Defekt in der Glasscheibe geschlossen wird.

In den Veröffentlichungen von Li Xiang et al. "Inspection and Image Analysis of Nickel Sulphide Inclusions in Toughened Glass Panels", CONTROL, AUTOmATION, ROBOTICS AND VISION, 2006, ICARCV '06. 9th International Conference IEEE, 5. Dezember 2006, Seiten 1-6, ISBN: 978-1-4244-0341-7, sowie Li Xiang et al. "In-Situ Inspection of Inclusions in Toughened Glass Panels of High-Rise Buildings", vol 10, no. 4, 30. April 2005, Seiten 1/7 - 7/7, XP002742907, NDT.net, werden ein Verfahren und eine Vorrichtung zur Detektion von Nickelsulfid-Einschlüssen beschrieben. Dabei wird Licht seitlich in die zu überprüfende Glasscheibe eingekoppelt und durch Totalreflektion über die gesamte überprüfbare Fläche der Glasscheibe verteilt, um bei Einschlüssen Streulicht zu erzeugen, welches im Verlauf eines Detektionsschritts mit einer ersten Detektoreinrichtung nachgewiesen wird. Danach wird mit einem Mikroskop in dem Tiefenermittlungsschritt die Tiefe des Einschlusses innerhalb der Glasscheibe ermittelt. Anschließend werden anhand einer stark vergrößerten Aufnahme des Einschlusses mit dem Mikroskop oder mit anderen Messverfahren Nickel-Sulfid-Einschlüsse identifiziert.

Es wird als eine Aufgabe der vorliegenden Erfindung angesehen, eventuell in einer Glasplatte befindliche Nickelsulfid-Einschlüsse möglichst schnell, kostengünstig und zuverlässig zu erkennen, damit durch geeignete Maßnahmen das Risiko und Gefährdungspotenzial eines durch einen Nickelsulfid-Einschluss verursachten Spontanbruchs der Glasplatte reduziert und nach Möglichkeit vollständig ausgeschlossen werden kann.

Diese Aufgabe wird erfindungsgemäß durch ein Verfahren gemäß Anspruch 1 und durch eine Vorrichtung gemäß Anspruch gelöst. Überraschenderweise wurde festgestellt, dass sich die in der Glasplatte vorhandenen Nickelsulfid-Einschlüsse zerstörungsfrei durch eine Analyse einer geeigneten Auswahl von mehreren charakteristischen Eigenschaften eindeutig bzw. mit einer äußerst geringen Fehlererwartung identifizieren und erkennen lassen. Eine zerstörungsfreie Analyse kann beispielsweise durch eine Auswertung von optischen Aufnahmen erfolgen. Eine hierfür geeignete optische Ausnahme kann dabei eine vollständige oder bereichsweise erzeugte Abbildung der Glasplatte sein, die mit einer bildgebenden Einrichtung erzeugt wurde. Es ist ebenfalls möglich, eine spektroskopische Analyse eines Emissions- oder Transmissionsspektrums einer optischen Aufnahme durchzuführen. Charakteristische Eigenschaften können auch eine lokale Änderung des Brechungsindex im Umfeld des zu überprüfenden Einschlusses, das Reflexionsverhalten im Bereich des Einschlusses oder das Vorhandensein eines mechanischen Spannungsfeldes im Umfeld des Einschlusses sein. Zu allen charakteristischen Eigenschaften, die für die Analyse und die Erkennung von Nickelsulfid-Einschlüssen geeignet sind und verwendet werden können, können jeweils erste oder zweite Kriterien vorgegeben werden, die überprüft werden können, um einen Nickelsulfid-Einschluss zu identifizieren und von anderen Fehlstellen auf oder in der Glasplatte zu unterscheiden.

Mit dem ersten Kriterium soll lediglich eine rasche Identifikation möglicher Einschlüsse in der Glasplatte erfolgen können. Hierfür können bereits beliebige geeignete Eigenschaften wie beispielsweise eine auffällige lokale Verfärbung in der optischen Aufnahme der Glasplatte verwendet werden. Die weiteren Untersuchungen und Überprüfungen werden auf die Bereiche mit möglichen Einschlüssen konzentriert, die mittels der ersten Kriterien identifiziert wurden.

Es hat sich gezeigt, dass eine für praktische Anwendungen ausreichend präzise Erkennung von Nickelsulfid-Einschlüssen bereits durch die Analyse bzw. Auswertung von mindestens zwei geeignet ausgewählten charakteristischen Eigenschaften erfolgen kann. Es hat sich beispielsweise herausgestellt, dass die Größe und die Formgebung von Nickelsulfid-Einschlüssen zwei charakteristische Eigenschaften darstellen, die eine rasche und zuverlässige Erkennung von Nickelsulfid-Einschlüssen ermöglichen.

Es können jedoch auch zwei andere Eigenschaften oder mehrere charakteristische Eigenschaften anhand der optischen Aufnahme ausgewertet und mittels geeignet vorgegebenen zweiten Kriterien festgestellt werden, ob es sich um Nickelsulfid-Einschlüsse handelt. Dabei wird bei einer Verwendung von mehreren oder jeweils aufwändig analysierbaren charakteristischen Eigenschaften die Anzahl von fehlerhaft erkannten oder nicht erkannten Nickelsulfid-Einschlüssen reduziert werden können, jedoch mehr Zeit und Kosten für die Durchführung des erfindungsgemäßen Verfahrens erforderlich werden. Gegebenenfalls können zwei geeignet ausgewählte charakteristische Eigenschaften der Einschlüsse auch ohne die Ermittlung der Tiefe des jeweiligen Einschlusses ausgewertet und für eine ausreichend aussagekräftige Erkennung von Nickelsulfid-Einschlüssen verwendet werden.

Durch die Ermittlung der Tiefe des möglichen Einschlusses in der Glasplatte und die anschließende Auswertung einer auf die ermittelte Tiefe fokussierten optischen Aufnahme wird erfindungsgemäß erreicht, dass das Risiko einer fehlerhaften Auswertung auf Grund von Abbildungsfehlern, einer eventuellen Unschärfe oder einer zu geringen Auflösung der für die Auswertung verwendeten optischen Aufnahme erheblich reduziert und dadurch die Genauigkeit bei der Identifizierung von Nickelsulfid-Einschlüssen gesteigert werden kann.

Es ist grundsätzlich denkbar, dass während des Tiefenermittlungsschritts bereits mehrere auf unterschiedliche Tiefenebenen der Glasplatte fokussierte optische Aufnahmen angefertigt werden und beispielsweise anhand der Konturenschärfe oder des Kontrastes eines in den Abbildungen sichtbaren Einschlusses die Tiefe des möglichen Einschlusses ermittelt wird, so dass in den meisten Fällen bereits während des Tiefenermittlungsschritts eine auf die anschließend ermittelte Tiefe ausreichend gut fokussierte optische Aufnahme erzeugt wird und für eine anschließende Auswertung zur Verfügung steht.

Die Bestimmung der Position des möglichen Einschlusses in der Glasplatte kann auf verschiedene Weisen erfolgen. Erfindungsgemäß ist vorgesehen, die Tiefe des möglichen Einschlusses durch zwei bildgebende Einrichtungen wie beispielsweise zwei Zeilenkameras oder zwei Digitalkameras oder eine Kombination von Zeilenkameras und Digitalkameras zu ermitteln, die aus unterschiedlichen Blickrichtungen den möglichen Einschluss abbilden und aus einem seitlichen Versatz des abgebildeten Einschlusses dessen Tiefe berechnen lassen.

In vielen Fällen kann eine einzige bildgebende Einrichtung für die Aufnahme der verschiedenen Aufnahmen verwendet werden, die für das erfindungsgemäße Verfahren ausgewertet werden. Es ist ebenfalls denkbar und im Hinblick auf eine möglichst kurze Verfahrensdauer zweckmäßig, mehrere Bildaufnahmeeinrichtungen bzw. eine größere Anzahl von Bildaufnahmeeinrichtungen zu verwenden, die jeweils nur für einen Bereich der Glasplatte zuständig sind und gegebenenfalls gleichzeitig mehrere verschiedene Aufnahmen aufnehmen können.

Es ist insbesondere im Hinblick auf einige Analyseverfahren und Auswertungsschritte von bestimmten charakteristischen Eigenschaften eines Einschlusses zweckmäßig, dass die auf die ermittelte Tiefe fokussierte optische Aufnahme nach dem Tiefenermittlungsschritt gesondert aufgenommen wird. In Kenntnis der ermittelten Tiefe des noch näher zu überprüfenden Einschlusses kann eine einzelne optische Aufnahme ausreichen, die den zu überprüfenden Einschluss in ausreichend hoher Auflösung und mit zutreffender Fokussierung auf die ermittelte Tiefe des Einschlusses erfasst bzw. abbildet.

Vorzugsweise ist vorgesehen, dass die nach dem Tiefenermittlungsschritt aufgenommene optische Aufnahme eine höhere Auflösung als die vorausgegangene Aufnahme der Glasplatte während des Detektionsschritts aufweist. Auf diese Weise kann erreicht werden, dass für die in dem Auswerteschritt ausgewertete optische Aufnahme eine ausreichend hohe Auflösung insbesondere in einem Bereich um den zu überprüfenden Einschluss vorgegeben wird und die Auswertung dadurch erleichtert bzw. präzisiert werden kann, während in dem vorausgehenden Detektionsschritt möglichst rasch und effizient eine großflächige Überprüfung der Glasplatte vorgenommen werden kann, die lediglich dazu dient, mögliche Einschlusspositionen zu bestimmen, die anschließend näher überprüft und ausgewertet werden. Die konstruktiven Anforderungen sowie die Dauer und Kosten für die Durchführung des erfindungsgemäßen Verfahrens können dadurch in vorteilhafter Weise reduziert werden.

Gemäß einer vorteilhaften Ausgestaltung des erfindungsgemäßen Verfahrens ist vorgesehen, dass in dem Auswerteschritt ausgehend von einer auf die ermittelten Tiefe fokussierten optischen Aufnahme eine Formgebung des möglichen Einschlusses ausgewertet wird. Es hat sich gezeigt, dass Nickelsulfid-Einschlüsse eine charakteristische Formgebung aufweisen, die eine zuverlässige Überprüfung ermöglicht. Da die für die Auswertung der Formgebung verwendete optische Aufnahme auf die ermittelte Tiefe fokussiert ist, wird vermieden, dass Fehlstellen oder Verunreinigungen auf oder in der Glasplatte, die eine abweichende Formgebung aufweisen, auf Grund von einer nicht ausreichenden Tiefenschärfe bzw. auf Grund von Abbildungsfehlern in der optischen Aufnahme keine unterscheidungskräftige Formgebung aufweisen.

Gemäß einer besonders vorteilhaften Ausgestaltung des Erfindungsgedankens ist vorgesehen, dass anhand der auf die ermittelte Tiefe fokussierten optischen Aufnahme ein Durchmesser des möglichen Einschlusses ermittelt und in einem Auswahlschritt diejenigen möglichen Einschlusspositionen ausgewählt werden, deren ermittelter Durchmesser innerhalb eines vorgegebenen Bereichs liegt, und das in dem Auswerteschritt nur für die in dem Auswahlschritt ausgewählten möglichen Einschlusspositionen einer auf die ermittelte Tiefe fokussierte optische Aufnahme ausgewertet wird. Die charakteristische Größe von Nickelsulfid-Einschlüssen liegt üblicherweise zwischen 50 µm und 500 um. In einer Glasplatte können auch kleinere oder größere Nickelsulfid-Einschlüsse vorkommen. Das Risiko eines unangekündigten Spontanbruchs ist in der Praxis erfahrungsgemäß jedoch nur für Nickelsulfid-Einschlüsse mit einer Mindestgröße von 50 µm und mehr relevant.

Die Wahrscheinlichkeit, mit der ein Spontanbruch auftreten wird, hängt unter anderem von dem Durchmesser des Nickelsulfid-Einschlusses, von dessen Tiefe innerhalb der Glasplatte und von einer während des Herstellungsverfahrens der Glasplatte künstlich erzeugten thermischen Eigenspannung der Glasplatte ab. Je höher eine vorgegebene thermische Eigenspannung der Glasplatte ist, umso geringer kann der Durchmesser eines Nickelsulfid-Einschlusses sein, der im Hinblick auf das Risiko eines Spontanbruchs relevant ist. Es ist deshalb vorgesehen, dass in dem zweiten Kriterium bei der Auswertung der fokussierten optischen Aufnahme in dem Auswerteschritt der vorgegebene Bereich und insbesondere die Bereichsgrenzen für den Durchmesser eines Nickelsulfid-Einschlusses, der zu einer Kennzeichnung oder zu einem Aussortieren der betreffenden Glasplatte führt, an die thermische Eigenspannung der Glasplatte angepasst ist. Es ist ebenfalls möglich, einen vergleichsweise großen Bereich für den Durchmesser von Nickelsulfid-Einschlüssen vorzugeben, um das erfindungsgemäße Verfahren zur Erkennung der Nickelsulfid-Einschlüsse rasch und dahingehend zuverlässig durchzuführen, dass zumindest alle spontanbruchgefährdenden Nickelsulfid-Einschlüsse erkannt werden und gegebenenfalls in Kauf genommen wird, auch Glasplatten mit zu kleinen Nickelsulfid-Einschlüssen oder mit Fehlstellen, die aufgrund ihrer Größe keine Nickelsulfid-Einschlüsse sein können, als relevante Einschlüsse zu identifizieren und die Glasplatte entsprechend zu kennzeichnen oder auszusortieren.

Es ist ebenfalls möglich, dass anhand der auf die ermittelte Tiefe fokussierten optischen Aufnahme ein Durchmesser des möglichen Einschlusses ermittelt und in einem Auswahlschritt diejenigen möglichen Einschlusspositionen ausgewählt werden, deren Durchmesser zwischen einem unteren Schwellenwert und einem oberen Schwellenwert für den Durchmesser liegt, und nur für diejenigen möglichen Einschlusspositionen die Auswertung anhand von weiteren Kriterien fortgesetzt wird, deren Durchmesser zwischen einem unteren Schwellenwert und einem oberen Schwellenwert für den Durchmesser liegt. In diesem Fall wird das Kriterium des Durchmessers des Nickelsulfid-Einschlusses lediglich als ein weiteres Kriterium für eine Vorauswahl von möglichen Einschlüssen herangezogen, die anschließend im einzelnen mit einem weiteren Analyseverfahren überprüft und gegebenenfalls als Nickelsulfid-Einschluss erkannt werden.

Ein weiteres Kriterium, das für die Erkennung von möglicherweise problematischen Nickelsulfid-Einschlüssen verwendet werden kann, ist die Tiefe des Nickelsulfid-Einschlusses in der Glasplatte, bzw. eine Position des Einschlusses in eine Dickenrichtung der Glasplatte.

Es ist bekannt, dass eine thermisch vorgespannte Glasplatte in Dickenrichtung einen parabelförmigen Spannungsverlauf aufweist, wobei an den einander gegenüberliegenden Oberflächen (Oberseite und Unterseite der Glasplatte) auftretende Druckspannungen etwa doppelt so groß sind wie Zugspannungen innerhalb eines Kernbereichs um eine Mittenebene der Glasplatte. Eine thermisch vorgespannte Glasplatte besitzt üblicherweise Druckzonen an den Oberflächen der Glasplatte, die jeweils etwa 20% in die Glasplatte hineinragen, und eine Zugzone in dem Kernbereich der Glasplatte, die etwa 60% einer Dicke der Glasplatte umfasst. Durch die Ermittlung der Tiefe des Einschlusses in der Glasplatte kann festgestellt werden, ob der Einschluss in einer Druckzone in einem Randbereich an einer der beiden Oberflächen der Glasplatte positioniert ist, oder ob sich der Einschluss in der Zugzone in dem Kernbereich der thermisch vorgespannten Glasplatte befindet. Durch einen Nickelsulfid-Einschluss induzierte Spontanbrüche treten erfahrungsgemäß nur dann auf, wenn der Nickelsulfid-Einschluss in der Zugzone der thermisch vorgespannten Glasplatte liegt.

Es ist deshalb optional vorgesehen, dass in einem Auswahlschritt diejenigen möglichen Einschlusspositionen ausgewählt werden, deren ermittelte Tiefe innerhalb eines Kernbereichs um die Mittenebene der Glasplatte liegt, und dass in dem Auswerteschritt nur für die in dem Auswahlschritt ausgewählten möglichen Einschlusspositionen eine auf die ermittelte Tiefe fokussierte optische Aufnahme ausgewertet wird. Auf diese Weise kann ein weiterer Vorteil des erfindungsgemäßen Verfahrens erreicht werden, demzufolge nur diejenigen Nickelsulfid-Einschlüsse für die weitere Auswertung und gegebenenfalls eine Kennzeichnung oder das Aussortieren der Glasplatte herangezogen werden, die innerhalb der Zugzone liegen und Spontanbrüche induzieren können. Dagegen werden beliebige Einschlüsse einschließlich Nickelsulfid-Einschlüsse, die in der für das Risiko eines Spontanbruchs nicht relevanten Druckzone der thermisch vorgespannten Glasplatte liegen, bei der erfindungsgemäßen Auswertung und Erkennung von möglicherweise relevanten Nickelsulfid-Einschlüssen nicht weiter berücksichtigt. Durch diese Vorselektion der möglichen Einschlüsse kann die für die Überprüfung einer Glasplatte erforderliche Zeit wesentlich verkürzt werden, da eine umfassende Überprüfung von Einschlüssen nur an einer geringen Anzahl von bereits vorselektierten Einschlüssen, bzw. Einschlusspositionen erforderlich ist und durchgeführt werden muss.

In vielen Fällen ist es vorteilhaft, mit dem erfindungsgemäßen Verfahren und einer hierfür geeigneten Vorrichtung ein zweistufiges Erkennungsverfahren durchzuführen. In einer ersten Verfahrensstufe wird eine das gesamte Volumen der Glasplatte umfassende vollflächige Überprüfung der Glasplatte vorgenommen. In der ersten Verfahrensstufe wird in dem Detektionsschritt mit einer optischen Bildaufnahmeeinrichtung eine Aufnahme mit einer üblicherweise geringen Vergrößerung bzw. mit einer vergleichsweise geringen, bzw. groben Auflösung erzeugt. Mit ersten Kriterien können bereits diejenigen charakteristischen Eigenschaften überprüft und anhand der ersten Kriterien eine Auswahl möglicher Einschlüsse vorgenommen werden, deren Analyse mit der bereits erzeugten optischen Aufnahme zuverlässig möglich ist, wodurch eine Selektion der in dem Detektionsschritt detektierten möglichen Einschlüsse erfolgt. Als erstes Kriterium könnte dabei beispielsweise die Größe des möglichen Einschlusses herangezogen und ausgewertet werden.

Anschließend wird für die nicht bereits ausgeschlossenen Einschlüsse deren Tiefe in der Glasplatte ermittelt.

In einer nachfolgenden Verfahrensstufe werden die als relevant erachteten möglichen Einschlüsse einer weiteren Analyse und Auswertung unterzogen. Dabei wird üblicherweise eine erneute optische Abbildung oder Analyse mit einer hohen Auflösung vorgenommen. Da die Tiefe des Einschlusses in der Glasplatte bereits bekannt ist, kann eine hochaufgelöste und konturenscharfe Abbildung oder optische Analyse des Einschlusses erzeugt werden, die eine zuverlässige und sehr präzise Erkennung von Nickelsulfid-Einschlüssen ermöglicht.

In dem Auswerteschritt kann dabei mindestens eine der nachfolgend genannten charakteristischen Eigenschaften des möglichen Einschlusses ermittelt und überprüft werden: eine Größe oder ein Durchmesser des Einschlusses, eine Form des Einschlusses, die Oberflächenstruktur des Einschlusses, das Vorhandensein von mechanischen Spannungen in einem Bereich um den Einschluss, eine ortsaufgelöste spektroskopische Abbildung des Einschlusses, eine wellenlängenabhängige Reflexion des beleuchteten Einschlusses, oder ein wellenlängenabhängiger Brechungsindex des Einschlusses sowie eines Bereichs um den Einschluss.

In einem nachfolgend exemplarisch dargestellten Ausführungsbeispiel wird ein möglicher Verfahrensablauf für eine Selektion und Detektion von Glasfehlern in Floatglas beschrieben. In einer Rohglastafel mit einer Dicke von 12 mm wird mit einer Selektoreinrichtung mit Hilfe einer bildgebenden Aufnahmeeinrichtung ein Einschluss in der Tiefe von 6 mm erkannt und die Position des Einschlusses hinsichtlich seiner Flächenkoordinaten und der Tiefenlage in der Rohglastafel bestimmt. Die Koordinaten des Einschlusses werden einer Detektoreinrichtung übergeben. Diese fährt die Position des Einschlusses an und untersucht den Einschluss mit den vorgegebenen Methoden der Spektroskopie, Spannungsoptik und Fotografie. Im Ergebnis zeigt sich, dass der Einschluss eine starke Absorption (Optische Dichte > 3) im Wellenlängenbereich 400 nm bis 800 nm aufweist und mechanische Spannungen im Umfeld des Einschlusses nachweisbar sind. Die fotografische Ermittlung der äußeren Form des Einschlusses zeigt eine gleichmäßige Oberfläche des Einschlusses, bei gleichzeitig elliptischer geometrischer Form. Anhand der drei Merkmale aus spektroskopischer, spannungsoptischer und fotografischer Messung kann der Einschluss zuverlässig als Nickelsulfid-Einschluss erkannt und identifiziert werden. Da er sich im Inneren des Glases befindet, ist er als Gefährdung für die Qualität des Glasproduktes bei der Weiterverarbeitung zu thermisch vorgespanntem Sicherheitsglas einzustufen.

Das vorangehend beschriebene Verfahren zur Erkennung von Nickelsulfid-Einschlüssen kann mit den meisten Glasherstellungsverfahren kombiniert und bereits während der Herstellung der Glasplatten durchgeführt werden. Dabei können die erforderlichen optischen Aufnahmen und Untersuchungen gegebenenfalls in den Herstellungsprozess eingebunden und beispielsweise noch an einem Glasplatten-Endlosband innerhalb eines üblichen Floatprozesses, noch bevor das Glasplatten-Endlosband zu einzelnen Glasscheiben zertrennt wird, durchgeführt werden. Auch lässt sich das Verfahren sowohl an vertikalen als auch an horizontalen Anlagen oder Transportstrecken einsetzen.

Nachfolgend wird in der exemplarischen Abbildung ein Ausführungsbeispiel des Erfindungsgedankens näher beschrieben. Es zeigt:

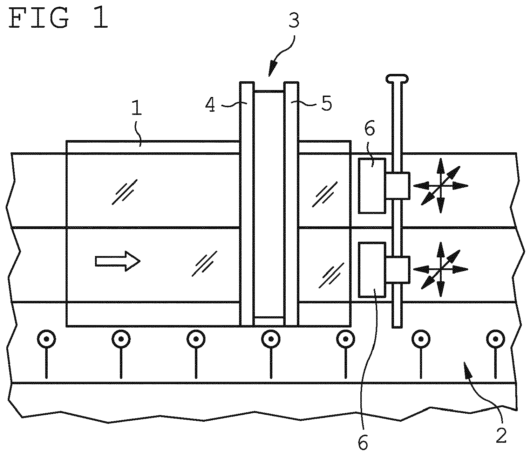

Fig. 1 eine schematische Darstellung einer Vorrichtung zur Erkennung von Nickelsulfid-Einschlüssen in einer Glasplatte.

In Fig. 1 wird ein Beispiel für die Vorrichtung zur Selektion und Detektion von Glasfehlern in einer Glasscheibe aus Floatglas beschrieben. Die Vorrichtung ist in eine Vertikalförderlinie zur Verabeitung von Rohglastafeln nach einem Waschprozess integriert, mit welchem eine Reinigung von Oberflächen der Floatglasscheibe durchgeführt wurde.

Die Glasscheibe 1 wird in der Vertikalförderlinie 2 einer Selektoreinrichtung 3 zugeführt. Die Selektoreinrichtung 3 weist zwei ortsfeste Kamerabalken 4 und 5 mit einer Durchlicht-Beleuchtung auf, wobei ein erster Kamerabalken 4 senkrecht und ein zweiter Kamerabalken 5 in einem Winkel geneigt zu der Glasscheibe 1 angeordnet ist. Mit der Selektoreinrichtung 3 werden digitale Bilder der in dem gezeigten Ausführungsbeispiel von links nach rechts geförderten Glasscheibe 1 erzeugt. Diese digitalen Bilder werden in Echtzeit hinsichtlich der Größe, Form, Intensität und Lage von Glasfehlern in Tiefenrichtung ausgewertet, um potentielle Nickelsulfid-Einschlüsse zu selektieren. Die vorselektierten Glasfehler werden von einer der zwei senkrecht zur Förderrichtung der Glasscheibe 1 und in Tiefenrichtung der Glasscheibe 1 beweglichen Zeilenkameras 6 mit einer Durchlicht-Beleuchtung angefahren und mit höherer Auflösung aufgenommen. Die beweglichen Zeilenkameras 6 bilden einen Detektor, mit dem einzelne Bereiche der Glasscheibe 1 hinsichtlich verschiedener charakteristischer Eigenschaften und Kriterien im Detail überprüft werden können. Durch eine Auswertung hinsichtlich der Intensität, der Form und der Oberflächenstruktur des Glasfehlers werden potentiell gefährliche Nickelsulfid-Einschlüsse erkannt.

## Patentansprüche

1. Verfahren zum Erkennen von Nickelsulfid-Einschlüssen in einer Glasplatte (1), wobei in einem Detektionsschritt mit einer optischen Bildaufnahmeeinrichtung (3) mindestens eine Aufnahme von mindestens einem Bereich der Glasplatte (1) aufgenommen und anhand von mindestens einem vorgegebenen ersten Kriterium mögliche Einschlusspositionen automatisiert bestimmt werden, wobei in einem Tiefenermittlungsschritt eine Tiefe des möglichen Einschlusses in der Glasplatte (1) ermittelt wird, und wobei in einem nachfolgenden Auswerteschritt eine auf die ermittelte Tiefe fokussierte optische Aufnahme anhand von mindestens einem vorgegebenen zweiten Kriterium ausgewertet wird, um ausgehend von dem möglichen Einschluss einen Nickelsulfid-Einschluss zu erkennen, **dadurch gekennzeichnet, dass** mit einer Selektoreinrichtung (3) mit zwei ortsfesten Kamerabalken (4, 5) digitale Bilder aufgenommen werden, wobei ein erster Kamerabalken (4) senkrecht und ein zweiter Kamerabalken (5) in einem Winkel geneigt zu der Glasscheibe (1) angeordnet ist, sodass die Tiefe des möglichen Einschlusses durch zwei Abbildungen aus unterschiedlichen Blickrichtungen oder durch eine optische Aufnahme, die in einem Winkel zu einer Lotrechten zur Glasplatte aufgenommen wurde, ermittelt werden kann, dass vorselektierte Glasfehler von einer von zwei senkrecht zur Förderrichtung der Glasscheibe (1) und in Tiefenrichtung der Glasscheibe (1) beweglichen Zeilenkameras (6) mit einer Durchlicht-Beleuchtung angefahren und mit höherer Auflösung aufgenommen werden, und dass der Detektionsschritt und der Tiefenermittlungsschritt jeweils mit einer Durchlicht-Beleuchtung durchgeführt werden.

2. Verfahren nach Anspruch 1, **dadurch gekennzeichnet, dass** die auf die ermittelte Tiefe fokussierte optische Aufnahme nach dem Tiefenermittlungsschritt aufgenommen wird.

3. Verfahren nach Anspruch 2, **dadurch gekennzeichnet, dass** die nach dem Tiefenermittlungsschritt aufgenommene optische Aufnahme eine höhere Auflösung als die vorausgegangene Aufnahme der Glasplatte (1) während des Detektionsschritts aufweist.

4. Verfahren nach einem der Ansprüche 1 bis 3, **dadurch gekennzeichnet, dass** in dem Auswerteschritt ausgehend von einer auf die ermittelte Tiefe fokussierten optischen Aufnahme eine Formgebung des möglichen Einschlusses ausgewertet wird.

5. Verfahren nach einem der vorangehenden Ansprüche, **dadurch gekennzeichnet, dass** in einem Auswahlschritt diejenigen möglichen Einschlusspositionen ausgewählt werden, deren ermittelte Tiefe innerhalb eines Kernbereichs um die Mittenebene der Glasplatte (1) liegt, und dass in dem Auswerteschritt nur für die in dem Auswahlschritt ausgewählten möglichen Einschlusspositionen eine auf die ermittelte Tiefe fokussierte optische Aufnahme ausgewertet wird.

6. Verfahren nach Anspruch 5, **dadurch gekennzeichnet, dass** in dem Auswahlschritt diejenigen möglichen Einschlusspositionen ausgewählt werden, deren ermittelte Tiefe außerhalb von vorgegebenen und an die beiden gegenüberliegenden Oberflächen der Glasplatte (1) jeweils angrenzenden Randbereiche der Glasplatte (1) liegt, und dass in dem Auswerteschritt nur für die in dem Auswahlschritt ausgewählten möglichen Einschlusspositionen eine auf die ermittelte Tiefe fokussierte optische Aufnahme ausgewertet wird.

7. Verfahren nach einem der vorangehenden Ansprüche, **dadurch gekennzeichnet, dass** anhand der auf die ermittelte Tiefe fokussierten optischen Aufnahme ein Durchmesser des möglichen Einschlusses ermittelt und in einem Auswahlschritt diejenigen möglichen Einschlusspositionen ausgewählt werden, deren ermittelter Durchmesser innerhalb eines vorgegebenen Bereichs liegt, und dass in dem Auswerteschritt nur für die in dem Auswahlschritt ausgewählten möglichen Einschlusspositionen eine auf die ermittelte Tiefe fokussierte optische Aufnahme ausgewertet wird.

8. Verfahren nach einem der vorangehenden Ansprüche, **dadurch gekennzeichnet, dass** in dem Auswerteschritt mindestens eine der nachfolgend genannten charakteristischen Eigenschaften des möglichen Einschlusses ermittelt und überprüft werden: eine Größe oder ein Durchmesser des Einschlusses, eine Form des Einschlusses, die Oberflächenstruktur des Einschlusses, das Vorhandensein von mechanischen Spannungen in einem Bereich um den Einschluss, eine ortsaufgelöste spektroskopische Abbildung des Einschlusses, eine wellenlängenabhängige Reflexion des beleuchteten Einschlusses, oder ein wellenlängenabhängiger Brechungsindex des Einschlusses sowie eines Bereichs um den Einschluss.

9. Vorrichtung zum Erkennen von Nickelsulfid-Einschlüssen in einer Glasplatte (1), wobei die Vorrichtung eine optische Bildaufnahmeeinrichtung (4, 5, 6, 7) aufweist, mit der eine Aufnahme von mindestens einem Bereich der Glasplatte (1) aufgenommen werden kann, wobei die Vorrichtung eine Einrichtung zur Ermittlung einer Tiefe eines Einschlusses in der Glasplatte (1) aufweist, und wobei die Vorrichtung eine Auswerteeinrichtung aufweist, mit der eine auf die ermittelte Tiefe fokussierte optische Aufnahme ausgewertet werden kann, um mit einem Verfahren gemäß einem der Ansprüche 1 bis 8 ausgehend von einem möglichen Einschluss einen Nickelsulfid-Einschluss zu erkennen, **dadurch gekennzeichnet, dass** die Vorrichtung eine Selektoreinrichtung (3) mit zwei ortsfesten Kamerabalken (4, 5) aufweist, wobei ein erster Kamerabalken (4) senkrecht und ein zweiter Kamerabalken (5) in einem Winkel geneigt zu der Glasscheibe (1) angeordnet ist, sodass die Tiefe des möglichen Einschlusses durch zwei Abbildungen aus unterschiedlichen Blickrichtungen oder durch eine optische Aufnahme, die in einem Winkel zu einer Lotrechten zur Glasplatte aufgenommen wurde, ermittelt werden kann, dass die Vorrichtung ferner zwei senkrecht zur Förderrichtung der Glasscheibe (1) und in Tiefenrichtung der Glasscheibe (1) bewegliche Zeilenkameras(6) aufweist, die dafür eingerichtet sind, dass vorselektierte Glasfehler von einer der zwei beweglichen Zeilenkameras (6) mit einer Durchlicht-Beleuchtung angefahren und mit höherer Auflösung aufgenommen werden können, und dass die Vorrichtung eine Durchlicht-Beleuchtung zur Durchführung des Detektionsschritts und des Tiefenermittlungsschritts aufweist.

## Claims

1. Method for detecting nickel sulphide inclusions in a glass plate (1), wherein in a detection step at least one recording of at least one region of the glass plate (1) is made with an optical image recording device (3) and positions of possible inclusions are automatically determined on the basis of at least one predetermined first criterion, wherein in a depth-determining step a depth of the possible inclusion in the glass plate (1) is determined, and wherein in a subsequent evaluation step an optical recording focused at the determined depth is evaluated on the basis of at least one predetermined second criterion, in order to detect from the possible inclusion a nickel sulphide inclusion, **characterised in that** digital images are recorded with a selector device (3) with two stationary camera beams (4, 5), wherein a first camera beam (4) is arranged perpendicularly and a second camera beam (5) is arranged inclined at an angle to the glass plate (1), such that the depth of the possible inclusion can be determined by two images from different viewing directions or by an optical image taken at an angle to a perpendicular to the glass plate, **in that** preselected glass defects are approached by one of two line cameras (6), which can be moved perpendicularly to the conveying direction of the glass plate (1) and in the depth direction of the glass plate (1), with transmitted-light illumination and are recorded with a higher resolution, and **in that** the detection step and the depth-determining step are each carried out with a transmitted-light illumination.

2. Method according to claim 1, **characterised in that** the optical recording focused at the determined depth is made after the depth-determining step.

3. Method according to claim 2, **characterised in that** the optical recording made after the depth-determining step has a higher resolution than the previous recording of the glass plate (1) during the detection step.

4. Method according to any one of claims 1 to 3, **characterised in that** in the evaluation step, starting from an optical image focused on the determined depth, a shaping of the possible inclusion is evaluated.

5. Method according to any one of the preceding claims, **characterised in that**, in a selection step, the possible inclusion positions are selected whose determined depth lies within a core region around the central plane of the glass plate (1), and **in that**, in the evaluation step, an optical image focused at the determined depth is evaluated only for the possible inclusion positions selected in the selection step.

6. Method according to claim 5, **characterised in that**, in the selection step, the possible inclusion positions are selected whose determined depth lies outside of predetermined edge regions of the glass plate (1) that are adjacent to the two opposite surfaces of the glass plate (1), and **in that**, in the evaluation step, an optical image focused at the determined depth is evaluated only for the possible inclusion positions selected in the selection step.

7. Method according to any one of the preceding claims, **characterised in that** a diameter of the possible inclusion is determined on the basis of the optical image focused on the determined depth and, in a selection step, the possible inclusion positions are selected whose determined diameter lies within a predetermined region, and **in that**, in the evaluation step, an optical image focused at the determined depth is evaluated only for the possible inclusion positions selected in the selection step.

8. Method according to any one of the preceding claims, **characterised in that**, in the evaluation step, at least one of the following characteristic properties of the possible inclusion is determined and verified: a size or a diameter of the inclusion, a shape of the inclusion, the surface structure of the inclusion, the presence of mechanical stresses in a region around the inclusion, a spatially resolved spectroscopic image of the inclusion, a wavelength-dependent reflection of the illuminated inclusion, or a wavelength-dependent refractive index of the inclusion and of a region around the inclusion.

9. Device for detecting nickel sulphide inclusions in a glass plate (1), wherein the device comprises an optical image recording device (4, 5, 6, 7) with which a recording of at least one region of the glass plate (1) can be made, wherein the device comprises a device for determining a depth of an inclusion in the glass plate (1), and wherein the device comprises an evaluation device with which an optical recording focused at the determined depth can be evaluated, in order to detect a nickel sulphide inclusion by means of a method according to any one of claims 1 to 8, starting from a possible inclusion, **characterised in that** the device comprises a selector device (3) with two fixed camera beams (4, 5), wherein a first camera beam (4) is arranged perpendicularly and a second camera beam (5) is arranged inclined at an angle to the glass plate (1), such that the depth of the possible inclusion can be determined by two images from different viewing directions or by an optical image taken at an angle to a perpendicular to the glass plate, **in that** the device further comprises two line cameras (6), which can be moved perpendicularly to the conveying direction of the glass plate (1) and in the depth direction of the glass plate (1), which are arranged therefor, **in that** preselected glass defects can be approached by one of the two movable line cameras (6) with transmitted-light illumination and recorded with higher resolution, and **in that** the device comprises a transmitted-light illumination for carrying out the detection step and the depth-determining step.

## Revendications

1. Procédé permettant de détecter des inclusions de sulfure de nickel dans une plaque de verre (1), dans lequel, dans une étape de détection, au moins une prise de vue d'au moins une zone de la plaque de verre (1) est effectuée avec une installation de prise de vue (3) optique et des emplacements d'inclusion possibles sont automatiquement déterminés à l'aide d'au moins un premier critère prédéfini, dans lequel, dans une étape de détermination de profondeur, une profondeur de l'inclusion possible dans la plaque de verre (1) est déterminée, et dans lequel, dans une étape suivante d'évaluation, une prise de vue optique réglée sur la profondeur déterminée est évaluée à l'aide d'au moins un second critère prédéfini pour détecter, à partir de l'inclusion possible, une inclusion de sulfure de nickel, **caractérisé en ce que** des images numériques sont enregistrées avec une installation de sélection (3) dotée de deux poutres de caméra (4, 5) fixes, une première poutre de caméra (4) étant disposée perpendiculairement et une seconde poutre de caméra (5) inclinée selon un angle par rapport à la plaque de verre (1), de telle sorte que la profondeur de l'inclusion possible peut être déterminée par deux images prises dans différentes directions de vision ou par une prise de vue optique qui a été effectuée selon un angle par rapport à une perpendiculaire à la plaque de verre, **en ce que** l'une de deux caméras à balayage linéaire (6) mobiles perpendiculairement à la direction de transport de la plaque de verre (1) et dans la direction de profondeur de la plaque de verre (1) s'approche de défauts du verre présélectionnés avec un éclairage par transmission et les photographie avec une résolution plus élevée, et **en ce que** l'étape de détection et l'étape de détermination de la profondeur sont exécutées respectivement avec un éclairage par transmission.

2. Procédé selon la revendication 1, **caractérisé en ce que** la prise de vue optique réglée sur la profondeur déterminée est effectuée après l'étape de détermination de profondeur.

3. Procédé selon la revendication 2, **caractérisé en ce que** la prise de vue optique effectuée après l'étape de détermination de profondeur présente une résolution plus élevée que la prise de vue précédente de la plaque de verre (1) effectuée pendant l'étape de détection.

4. Procédé selon l'une des revendications 1 à 3, **caractérisé en ce que**, dans l'étape d'évaluation, une forme de l'inclusion possible est évaluée à partir d'une prise de vue optique réglée sur la profondeur déterminée.

5. Procédé selon l'une des revendications précédentes, **caractérisé en ce que**, dans une étape de sélection, les emplacements d'inclusion possibles dont la profondeur déterminée est comprise dans une zone centrale autour du plan médian de la plaque de verre (1) sont sélectionnés, et **en ce que**, dans l'étape d'évaluation, une prise de vue optique réglée sur la profondeur déterminée est évaluée uniquement pour les emplacements d'inclusion possibles sélectionnés dans l'étape de sélection.

6. Procédé selon la revendication 5, **caractérisé en ce que**, dans l'étape de sélection, les emplacements d'inclusion possibles dont la profondeur déterminée se trouve en dehors de zones de bords de la plaque de verre (1) prédéfinies et respectivement adjacentes aux deux surfaces opposées de la plaque de verre (1) sont sélectionnés, et **en ce que**, dans l'étape d'évaluation, une prise de vue optique réglée sur la profondeur déterminée est évaluée uniquement pour les emplacements d'inclusion possibles sélectionnés dans l'étape de sélection.

7. Procédé selon l'une des revendications précédentes, **caractérisé en ce qu'**un diamètre de l'inclusion possible est déterminé à l'aide de la prise de vue optique réglée sur la profondeur déterminée et, dans une étape de sélection, les emplacements d'inclusion possibles dont le diamètre déterminé est compris dans une plage prédéfinie sont sélectionnés, et **en ce que**, dans l'étape d'évaluation, une prise de vue optique réglée sur la profondeur déterminée est évaluée uniquement pour les emplacements d'inclusion possibles sélectionnés dans l'étape de sélection.

8. Procédé selon l'une des revendications précédentes, **caractérisé en ce que**, dans l'étape d'évaluation, au moins une des propriétés caractéristiques de l'inclusion possible citées ci-après est déterminée et vérifiée : une grandeur ou un diamètre de l'inclusion, une forme de l'inclusion, la structure de surface de l'inclusion, la présence de tensions mécaniques dans une zone autour de l'inclusion, une image spectroscopique de l'inclusion avec résolution spatiale, un réfléchissement dépendant de la longueur d'onde de l'inclusion éclairée, ou un indice de réfraction dépendant de la longueur d'onde de l'inclusion ainsi que d'une zone autour de l'inclusion.

9. Dispositif permettant de détecter des inclusions de sulfure de nickel dans une plaque de verre (1), dans lequel le dispositif présente une installation de prise de vue (4, 5, 6, 7) optique, au moyen de laquelle une prise de vue d'au moins une zone de la plaque de verre (1) peut être effectuée, le dispositif présentant une installation permettant de déterminer une profondeur d'une inclusion dans la plaque de verre (1), et le dispositif présentant une installation d'évaluation, au moyen de laquelle une prise de vue optique réglée sur la profondeur déterminée peut être évaluée pour détecter une inclusion de sulfure de nickel à partir d'une inclusion possible au moyen d'un procédé selon l'une des revendications 1 à 8, **caractérisé en ce que** le dispositif présente une installation de sélection (3) dotée de deux poutres de caméra (4, 5) fixes, une première poutre de caméra (4) étant disposée perpendiculairement et une seconde poutre de caméra (5) inclinée selon un angle par rapport à la plaque de verre (1), de telle sorte que la profondeur de l'inclusion possible peut être déterminée par deux images prises dans différentes directions de vision ou par une prise de vue optique qui a été effectuée selon un angle par rapport à une perpendiculaire à la plaque de verre, **en ce que** le dispositif présente en outre deux caméras à balayage linéaire (6) mobiles perpendiculairement à la direction de transport de la plaque de verre (1) et dans la direction de profondeur de la plaque de verre (1), qui sont conçues pour que l'une des deux caméras à balayage linéaire (6) mobiles puisse s'approcher de défauts du verre présélectionnés avec un éclairage par transmission et les photographier avec une résolution plus élevée, et **en ce que** le dispositif présente un éclairage par transmission pour exécuter l'étape de détection et l'étape de détermination de profondeur.
